# EUROPEAN PATENT APPLICATION

(11) **EP 4 458 298 A1**
(43) Date of publication of application: **06.11.2024**
(21) Application number: 22914480.3
(22) Date of filing: 22.12.2022
(51) Int. Cl.: A61B 18/12, A61B 18/14

(54) **PUNCTURE SYSTEM AND CONTROL HANDLE THEREOF**

(30) Priority: 31.12.2021 CN 202111683250
(71) Applicant: Hangzhou Noya Medtech Co., Ltd., Hangzhou, Zhejiang 310000 (CN)
(72) Inventor: WANG, Yongsheng, Hangzhou, Zhejiang 310000 (CN)
(74) Representative: Mewburn Ellis LLP
(86) International application number: PCT/CN2022/141003
(87) International publication number: WO 2023/125226

(57) **Abstract**

A puncture system (800) and a control handle (3, 4, 5, 6, 7) of the puncture system (800) are provided. The control handle (3, 4, 5, 6, 7) includes a housing (41, 51, 61, 71) and a control mechanism. The housing (41, 51, 61, 71) defines an accommodation space (410) therein. The control mechanism includes an operation member (441, 541, 641, 741) attached to the housing (41, 51, 61, 71) and a control switch disposed in the accommodation space (410). As such, by manipulating the operation members (441, 541, 641, 741), the control switch can be switched between an on-state and an off-state. In the on-state, an electrode tip is electrically connected to an external power source. In the off-state, the electrode tip is electrically disconnected from the external power source, so that an operator can achieve the on/off state of the electric circuit by manipulating the operation members (441, 541, 641, 741), which is very convenient and facilitates improving the probability of the success rate of the puncture.

## Description

### CROSS-REFERENCE TO RELATED APPLICATION(S)

This application claims priority to and the benefit of Patent Application No. 202111683250.6, filed December 31, 2021 to China National Intellectual Property Administration (CNIPA), and entitled "PUNCTURE SYSTEM AND CONTROL HANDLE OF PUNCTURE SYSTEM", the entire disclosure of which is incorporated herein by reference.

### TECHNICAL FIELD

The disclosure relates to the field of medical device technologies, and in particular, to a puncture system and a control handle of the puncture system.

### BACKGROUND

Patients suffering from heart failure often experience an increase in pressure in the left atrium, which in turn leads to pulmonary congestion and severely affects the quality of life of the patients. Based on left ventricular ejection fraction (LVEF), heart failure can be classified into heart failure with reduced ejection fraction (HFrEF) and heart failure with preserved ejection fraction (HFpEF). Research results show that many drugs (such as angiotensin receptor blocker (ARB) and angiotensin converting enzyme inhibitor (ACEI)) which can improve prognosis and survival rate in HFrEF have no similar effect in HFpEF, making it difficult to improve symptoms of patients with HFpEF.

Atrial shunting, which establishes a shunt channel between atria to allow the right heart system to compensate for the volume load and thereby relieve the pressure load on the left heart system (particularly the left atrium), can directly improve the hemodynamic conditions of patients, reduce the mean left atrial pressure and pulmonary capillary wedge pressure, and thus has significant clinical importance in improving the symptoms and prognosis of patients with both HFrEF and HFpEF. The main methods include traditional transseptal puncture with balloon dilatation and implantation of devices to establish the shunt channel.

However, in the current puncture process, controlling the on-off of the power output of the puncture requires an assistant to operate a foot switch. The disadvantage of this method is that the assistant can only step on the foot switch after receiving instructions from the operator, which takes a certain amount of time. During this time period, the heartbeat may cause the puncture electrode to deviate from a target position, posing significant potential risks to the patient and having certain limitations.

### SUMMARY

The disclosure aims to provide a control handle that is hand-operable and a puncture system having the control handle, so as to solve a problem in the related art.

In order to solve the described technical problem, the disclosure adopts the following technical solutions. The control handle includes a housing and a control mechanism. The housing defines an accommodation space therein. The control mechanism includes an operation member attached to the housing and a control switch disposed in the accommodation space. The control switch is operable to be electrically connected to an electrode tip and an external power source. The operation member is configured to trigger the control switch to be in an on-state or an off-state. In the on-state, the electrode tip is electrically connected to the external power source, and in the off-state, the electrode tip is electrically disconnected from the external power source.

In some embodiments, the control switch includes a first connector, a second connector, a first cable, and a second cable. Both the first connector and the second connector are received in the accommodation space, and the first connector is spaced apart from the second connector. A distal end of the first cable is electrically connected to the first connector, a distal end of the second cable is electrically connected to the second connector, and both a proximal end of the first cable and a proximal end of the second cable are electrically connected to the external power source. The first connector is in contact with the second connector when the operation member abuts against the first connector and/or the second connector, and the control switch is in the on-state when the first connector is in contact with the second connector.

In some embodiments, the operation member includes a pressing portion configured to abut against the first connector and/or the second connector, the first connector is in contact with the second connector when the pressing portion abuts against the first connector and/or the second connector, and the control switch is in the on-state when the first connector is in contact with the second connector. The operation member is movable relative to the housing, and the operation member is configured to drive, under the action of an external force, the pressing portion to abut against the first connector and/or the second connector; alternatively, the operation member is fixed relative to the housing, and the control mechanism further includes a sensor disposed on the operation member and a drive member electrically connected to the sensor. The sensor is configured to generate a touch control signal in response to a preset touch control operation performed on the operation member, and the drive member is configured to drive the pressing portion to abut against the first connector and/or the second connector according to the touch control signal.

In some embodiments, the first connector and/or the second connector includes a triggering portion corresponding to the pressing portion. The pressing portion is configured to abut against the triggering portion to enable the triggering portion of the first connector to be in contact with the second connector, or to enable the triggering portion of the second connector to be in contact with the first connector, or to enable the triggering portion of the first connector to be in contact with the triggering portion of the second connector.

In some embodiments, the triggering portion is resilient, the triggering portion is deformable under the action of the external force, and is capable of restoring from deformation to reset when the external force is removed. Alternatively or additionally, the control mechanism further includes a reset member, where the reset member is disposed between the first connector and the second connector, and the reset member is configured to provide a force for resetting the triggering portion.

In some embodiments, the control mechanism further includes an elastic member configured to provide a force to enable the operation member to move away from the first connector.

In some embodiments, the control mechanism further includes a conductive member. A distal end of the conductive member extends out of the housing and is electrically connected to the electrode tip, and a proximal end of the conductive member is electrically connected to the control switch or the external power source.

In some embodiments, the proximal end of the conductive member is electrically connected to the first connector or the second connector; or the proximal end of the conductive member is electrically connected to the first cable or the second cable; or the proximal end of the conductive member is electrically connected to the external power source.

In some embodiments, the conductive member is configured as a conductive wire, where a distal end of the conductive wire is fixedly connected to the electrode tip; alternatively, the conductive member is configured as a conductive tube, where the conductive tube is integrally formed with the electrode tip, or the conductive tube is sleeved on the electrode tip, or the electrode tip is fixedly connected to a distal surface of the conductive tube.

In some embodiments, the operation member is movable relative to the housing in a radial direction of the housing.

In some embodiments, the first connector includes a connection piece and a first pin spaced apart from the connection piece. The second connector includes a base and a second pin. The base is disposed corresponding to the first connector and located at one side of the connection piece away from the operation member. The first pin and the second pin are arranged at intervals on the base, the first pin is electrically connected to the distal end of the first cable, and the second pin is electrically connected to the distal end of the second cable. Two contact points are disposed on the base. One of the two contact points is electrically connected to the first pin and the other of the two contact points is electrically connected to the second pin, and the triggering portion includes the connection piece. The operation member is configured to drive, under the action of an external force, the connection piece to be in contact with the two contact points, to enable the control switch to be in the on-state.

In some embodiments, the control mechanism further includes a fixing member. The fixing member is insulated from the first connector and the second connector, and the base is disposed in the accommodation space via the fixing member. The fixing member includes a fixing seat and multiple supporting legs supporting the fixing seat. The multiple supporting legs are fixed in the housing at intervals. The fixing seat defines a groove for receiving the base and through-holes for allowing the first pin and the second pin to extend through, where the through-holes are spaced apart from the multiple supporting legs, and both the first pin and the second pin extend out of the fixing seat.

In some embodiments, the triggering portion includes a free portion that is resilient. The free portion is disposed on the first connector, and a free end of the free portion is movable within the accommodation space. The second connector is located at one side of the first connector away from the operation member. The free portion is capable of driving, under an action of an external force, the free end of the free portion to move towards and be in contact with the second connector, to enable the control switch to be in the on-state.

In some embodiments, the first connector defines a through-hole at a middle portion of the first connector to form the free portion. The free portion extends in an axial direction of the housing.

In some embodiments, the control mechanism further includes a fixing member received in the accommodation space, where the fixing member is insulated from the first connector and the second connector. An axis of the fixing member is coaxial with an axis of the housing, the fixing member defines a through-hole and an accommodation groove, where the through-hole extends through both ends of the fixing member that are arranged along the axis of the fixing member, and the accommodation groove has an opening facing a proximal end, the first connector extends through the through-hole, and the second connector is disposed in the accommodation groove. A distal end of the accommodation groove extends to a position corresponding to the free portion and is in communication with the through-hole, so that the free portion is operable to be in contact with the second connector.

In some embodiments, the first connector includes a resilient piece and a conductive piece electrically connected to the resilient piece. Two ends of the conductive piece are both fixed to the resilient piece, the resilient piece is electrically connected to the first cable, the resilient piece defines a through-hole, the conductive piece is disposed corresponding to the through-hole of the resilient piece and between the resilient piece and the operation member, and the triggering portion includes the conductive piece. The second connector is disposed corresponding to the through-hole of the resilient piece and is electrically connected to the second cable, and the second connector is located at one side of the resilient piece away from the operation member. The operation member is configured to drive, under the action of an external force, a middle portion of the conductive piece to deform to be in contact with the second connector, to enable the control switch to be in the on-state.

In some embodiments, the conductive piece is arc-shaped, protrudes outwards towards the operation member, and extends in an axial direction of the housing. The second connector has a protruding portion configured to be electrically connected to the conductive piece, where the protruding portion extends in a circumferential direction of the housing.

In some embodiments, the control mechanism further includes a fixing member received in the accommodation space, where the fixing member is insulated from the first connector and the second connector. The fixing member extends in the axial direction. The fixing member is hollow to define an accommodation space for receiving the first connector and the second connector. The fixing member defines an opening, on a top of the fixing member, for allowing the pressing portion of the operation member to abut against and be in contact with the conductive piece, to enable the conductive piece to be in contact with the second connector.

In some embodiments, the fixing member defines a through-hole on a proximal end of the fixing member, a distal end of the resilient piece is connected to the fixing member via a fixing shaft, and a proximal end of the resilient piece extends through the through-hole of the fixing member. A proximal end of the second connector extends through the through-hole of the fixing member.

In some embodiments, the operation member is movable relative to the housing in an axial direction of the housing.

In some embodiments, the first connector has a first protrusion protruding towards the operation member, a proximal end of the first connector is electrically connected to the first cable, and a distal end of the first connector is electrically connected to the conductive member. The second connector has a second protrusion corresponding to the first protrusion, where a proximal end of the second connector is electrically connected to the second cable, and the triggering portion includes the first protrusion and the second protrusion. The first protrusion is disposed on a path along which the operation member is movable. When the operation member moves to a position where the first protrusion is located, the pressing portion of the operation member abuts against the first protrusion, so that the first protrusion is deformed to be in contact with the second protrusion to enable the control switch to be in the on-state.

In some embodiments, the second connector is in a tubular shape, and defines an opening for allowing the operation member to move. Fitting members are respectively sleeved on two ends of the first connector, and the second connector is sleeved on an outer periphery of each of the fitting members, so that the first connector is received in an inner cavity of the second connector, and the first connector is insulated from the second connector via the fitting members.

In some embodiments, the control handle includes a fluid supply mechanism. The fluid supply mechanism includes a fluid supply tubular member disposed at the middle of the housing, the fluid supply tubular member extends into the accommodation space in a direction towards a distal end of the housing, an outer periphery of the fluid supply tubular member and an inner wall of the housing cooperatively define a space for allowing both the first cable and the second cable to extend through. Both the first connector and the second connector are located at a distal end of the fluid supply tubular member and are spaced apart from the fluid supply tubular member, and the distal end of the fluid supply tubular member is in communication with a proximal end of a tube set extending into the accommodation space.

In some embodiments, the control handle includes a fluid supply mechanism. The fluid supply mechanism includes a fluid supply tubular member disposed at the middle of the housing. The fluid supply tubular member extends into the accommodation space and extends to a proximal end of the housing, and defines at a position corresponding to the operation member an opening for allowing the operation member to extend through, and the operation member is in interference fit with the fluid supply tubular member. The first connector and the second connector are received in the fluid supply tubular member, and part of the operation member is received in the fluid supply tubular member.

In some embodiments, the second connector is in snap-fit with the fluid supply tubular member; or a fixing member for fixing the first connector and the second connector is in snap-fit with the fluid supply tubular member.

In some embodiments, the control mechanism further includes a protective film that is insulating and waterproof. The protective film covers an outer periphery of the first connector and an outer periphery of the second connector and the operation member.

A puncture system is further provided in the disclosure. The puncture system includes an electrode tip, a tube set sleeved on the electrode tip, and the above-identified control handle, where a distal end of the control handle is fixedly connected to a proximal end of the tube set.

It can be seen from the described technical solutions that the disclosure has at least the following advantages and positive effects.

In the control handle provided in the disclosure, the control mechanism is attached to the housing, and the control mechanism includes the control switch and the operation member. As such, by manipulating the operation member, the control switch can be switched between the on-state and the off-state. In the on-state, the electrode tip is electrically connected to the external power source. In the off-state, the electrode tip is electrically disconnected from the external power source. Therefore, the operator can control on-off state of the electric circuit by manipulating the operation member, thereby achieving ablation puncture. Compared with an ablation puncture device in the related art where an on-off switch generally requires an assistant to operate a foot switch, which has the disadvantage that the assistant needs to wait for the operator's instructions before stepping on the foot switch, this process takes a certain time, and during this time period, the heartbeat may cause the puncture electrode to deviate from a target position, posing significant potential risks to the patient. By adding the control mechanism to the control handle, the operator can directly and immediately operate and control the control mechanism after aligning the position, which is very convenient and facilitates improving the success rate of the puncture.

### BRIEF DESCRIPTION OF THE DRAWINGS

FIG. 1 is a schematic structural view of a puncture system according to an embodiment of the disclosure.
FIG. 2 is a schematic structural view of a control handle according to a first embodiment of the disclosure.
FIG. 3 is a schematic structural view illustrating an interior of the control handle and an interior of part of a tube set according to the first embodiment of the disclosure.
FIG. 4 is a cross-sectional view of the control handle and part of the tube set according to the first embodiment of the disclosure.
FIG. 5 is a cross-sectional view illustrating the interior of the control handle according to the first embodiment of the disclosure.
FIG. 6 is a cross-sectional view illustrating the interior of the control handle according to the first embodiment of the disclosure, taken in another direction.
FIG. 7 is a schematic circuit diagram of the control handle according to the first embodiment of the disclosure.
FIG. 8 is a schematic structural view of a control handle according to a second embodiment of the disclosure.
FIG. 9 is a schematic structural view illustrating an interior of the control handle and an interior of part of a tube set according to the second embodiment of the disclosure, in which a power connector is not illustrated.
FIG. 10 is a partial cross-sectional view of the control handle according to the second embodiment of the disclosure.
FIG. 11 is a schematic structural view illustrating a first connector, a second connector, and a fixing member of the control handle according to the second embodiment of the disclosure.
FIG. 12 is a schematic structural view of a control handle according to a third embodiment of the disclosure.
FIG. 13 is a schematic structural view illustrating an interior of the control handle and an interior of part of a tube set according to the third embodiment of the disclosure, where a power connector is not illustrated.
FIG. 14 is a partial cross-sectional view of the control handle according to the third embodiment of the disclosure.
FIG. 15 is an exploded schematic structural view of a first connector, a second connector, and a fixing member of the control handle according to the third embodiment of the disclosure.
FIG. 16 is a schematic structural view illustrating an operation member, a first connector, and a second connector of the control handle according to the third embodiment of the disclosure, from one direction.
FIG. 17 is a schematic structural view illustrating the operation member, the first connector, and the second connector of the control handle according to the third embodiment of the disclosure, from another direction.
FIG. 18 is a schematic structural view of a control handle according to a fourth embodiment of the disclosure.
FIG. 19 is a schematic structural view illustrating an interior of the control handle and an interior of part of a tube set according to the fourth embodiment of the disclosure, where a power connector is not illustrated.
FIG. 20 is a partial cross-sectional view of the control handle according to the fourth embodiment of the disclosure.
FIG. 21 is an exploded schematic structural view of an operation member, a first connector, and a second connector of the control handle according to the fourth embodiment of the disclosure.

Reference numerals are descried as follows:
800. puncture system;
1. electrode tip; 2. tube set; 21. inner tube; 22. outer tube; 3. control handle;
4. control handle; 41. housing; 410. accommodation space; 411. upper cover; 412. lower housing; 42. power connector; 43. fluid supply mechanism; 431. fluid supply tubular member; 432. valve; 44. control mechanism; 440. control switch; 441. operation member; 442. conductive member; 443. first cable; 444. second cable; 445. first connector; 4451. connection piece; 4452. first pin; 446. second connector; 4461. base; 4463. contact point; 4464. groove; 4465. through-hole; 4466. channel; 4462. second pin; 447. fixing member; 4471. fixing seat; 4472. supporting leg; 448. pressing portion; 449. triggering portion; 46. front rubber sleeve;
91. first seat; 92. second seat;
5. control handle; 51. housing; 52. power connector; 53. fluid supply mechanism; 531. fluid supply tubular member; 532. valve; 540. control mechanism; 541. operation member; 542. conductive member; 543. first cable; 544. second cable; 545. first connector; 5451. distal connection portion; 5452. middle portion; 5453. proximal connection portion; 5454. free portion; 5450. through-hole; 546. second connector; 5461. distal connection rod; 5462. contact portion; 5463. proximal connection rod; 547. fixing member; 5471. through-hole; 5472. accommodation groove; 548. elastic member;
6. control handle; 61. housing; 62. power connector; 63. fluid supply mechanism; 631. fluid supply tubular member; 632. valve; 640. control mechanism; 641. operation member; 642. conductive member; 643. first cable; 644. second cable; 645. first connector; 6450. through-hole; 6451. resilient piece; 6452. conductive piece; 646. second connector; 6461. protruding portion; 6462. bending portion; 647. fixing member; 6470. accommodation space; 6471. opening; 6472. through-hole; 648. stopper; 649. fixing shaft;
7. control handle; 71. housing; 711. opening; 72. power connector; 73. fluid supply mechanism; 731. fluid supply tubular member; 732. valve; 741. operation member; 742. conductive member; 743. first cable; 744. second cable; 745. first connector; 7451. first protrusion; 746. second connector; 7461. second protrusion; 7462. opening; 747. fitting member.

### DETAILED DESCRIPTION

Exemplary embodiments that embody features and advantages of the disclosure will be described in detail hereinafter. It is to be understood that the disclosure may have various modifications in different embodiments without departing from the scope of the disclosure. The illustrations and drawings set forth herein are essentially for illustrative purposes and are not construed as limitations to the disclosure.

It may be understood that terms such as "center", "longitudinal", "lateral", "length", "width", "thickness", "upper", "lower", "front", "back", "left", "right", "vertical", "horizontal", "top", "bottom", "in", "out", "clockwise", "anticlockwise", and the like referred to herein which indicate directional relationship or positional relationship are directional relationship or positional relationship based on accompanying drawings and are only for the convenience of description and simplicity, rather than explicitly or implicitly indicate that apparatuses or components referred to herein must have a certain direction or be configured or operated in a certain direction and therefore cannot be understood as limitation to the disclosure. In addition, terms "first", "second", and the like are only used for description and cannot be understood as explicitly or implicitly indicating relative importance or implicitly indicating the number of technical features referred to herein. Therefore, features restricted by terms "first", "second", and the like can explicitly or implicitly include at least one of the features. In the context of the disclosure, unless stated otherwise, "multiple" refers to "at least two", such as two, three, and the like.

It may be noted that the puncture system and the control handle thereof in the disclosure may be suitable for situations including, but not limited to, transseptal puncture, and are also suitable for other tissues to be punctured. The use of the puncture system for an atrial septum is described and illustrated herein as an example.

The disclosure provides a puncture system, mainly used for puncture operations such as transseptal puncture. In the disclosure, when describing orientation, an operator of the puncture system is taken as a reference, one end closer to the operator is referred to as a proximal end, and an opposite end referred to as a distal end.

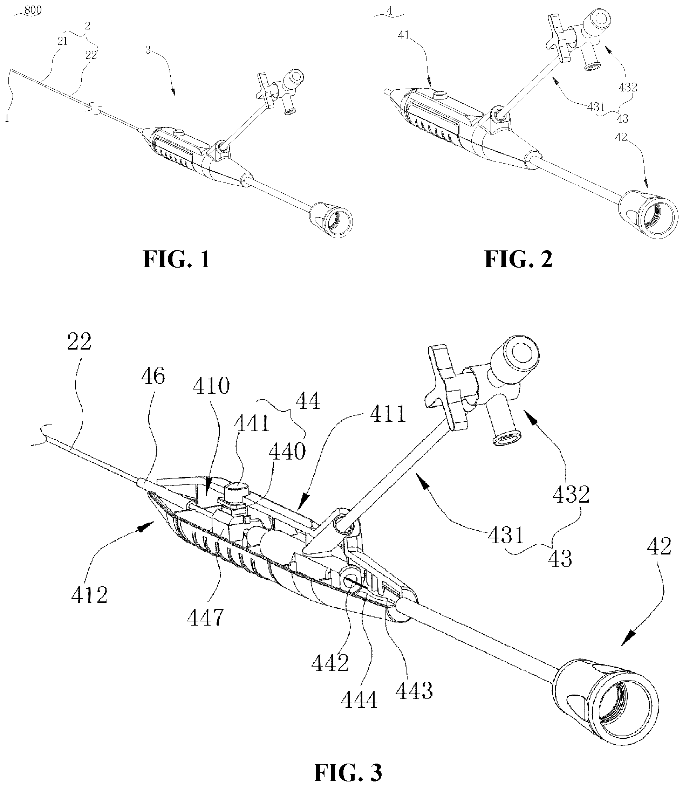

FIG. 1 is a schematic structural view of a puncture system according to an embodiment of the disclosure.

Referring to FIG. 1, a puncture system 800 includes an electrode tip 1, a tube set 2, and a control handle 3.

The tube set 2 includes an inner tube 21 and an outer tube 22 sleeved on an outer periphery of the inner tube 21.

The outer tube 22 mainly serves as a support. A material of the outer tube 22 includes, but is not limited to, at least one of polyether ether ketone (PEEK), polyether block polyamide (PEBAX), or nylon (PA).

A distal end of the inner tube 21 extends out of the outer tube 22, facilitating adjustment of a direction during puncture. The distal end of the inner tube 21 is sleeved on an outer periphery of the electrode tip 1, and is used for connecting the electrode tip 1. A material of the inner tube 21 includes, but is not limited to, at least one of PEBAX or PA, so that the inner tube 21 has good ductility.

The electrode tip 1 is disposed at a distal end of the tube set 2. Specifically, the inner tube 21 is sleeved on the electrode tip 1. Exemplarily, the electrode tip 1 is connected to the distal end of the inner tube 21 by heat melting.

A distal end of the electrode tip 1 is a solid atraumatic tip. A proximal end of the electrode tip 1 is a hollow tube. A developing block is provided in the hollow tube to fill in the vicinity of the tip, so as to facilitate tracking the position of the electrode tip 1 during an operation. A material of the electrode tip 1 is a conductive metal material.

A gap is defined between the outer periphery of the electrode tip 1 and an inner wall of the inner tube 21 to form a fluid channel for delivering a fluid, thereby allowing surgical substances such as heparin or contrast agent to be injected into a body during an operation. Exemplarily, both sides of the outer periphery of the electrode tip 1 may be symmetrically flatted to ensure even distribution of the contrast agent, thereby reducing a surgical risk. The both sides of the outer periphery of the electrode tip 1 may also be designed into a bidirectional concave structure, defining a fluid channel that allows a large amount of fluid to pass through, enabling rapid delivery of more contrast agents and making the operation more efficient. The electrode tip 1 may also be in the shape of a quadrangular prism, with corners adapted to the shape of the inner tube 21 to facilitate the connection between the inner tube 21 and the electrode tip 1. The electrode tip 1 may also be in a semicylindrical shape, allowing for a larger diameter of the fluid channel.

In other embodiments, the tube set 2 may also include a conductive tube covered with an insulating material. The conductive tube may be integrally formed with the electrode tip 1. Alternatively, the conductive tube may be sleeved on the electrode tip 1. Alternatively, the electrode tip 1 may be fixedly connected to a distal surface of the conductive tube.

In other embodiments, the puncture system may include, but is not limited to, puncture structures suitable such as puncture needles or puncture guide wires.

The control handle 3 is disposed at the proximal end of the tube set 2, and can be used for controlling the on-state and the off-state of a circuit, facilitating manipulation of an operator.

The control handle will be specifically described hereinafter through various embodiments.

### First Embodiment

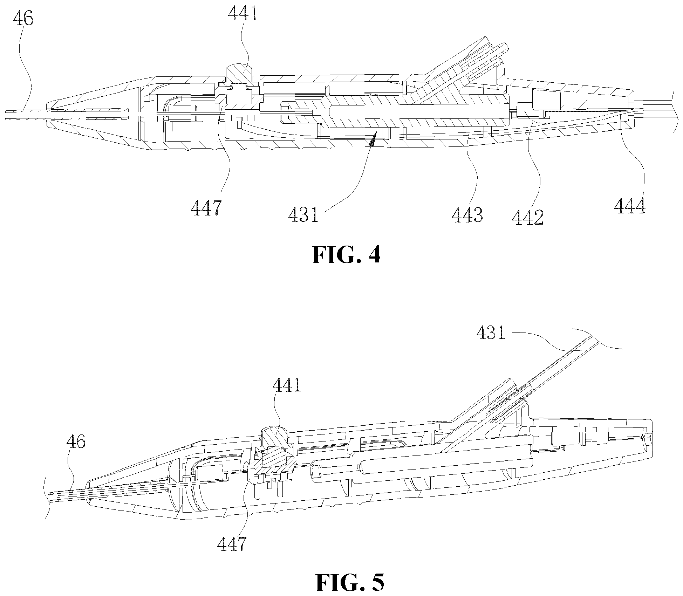

Referring to FIGS. 1 to 5 together, in the first embodiment of the disclosure, the control handle 4 includes a housing 41 and a control mechanism 44 attached to the housing 41. The housing 41 defines an accommodation space 410 therein. The control mechanism 44 includes a control switch 440 and an operation member 441. The operation member 441 is attached to the housing 41. The control switch 440 is disposed in the accommodation space 410. The control switch 440 is operable to be electrically connected to the electrode tip 1 and an external power source. The operation member 441 is configured to trigger the control switch 440 to be in an on-state or an off-state. In the on-state, the electrode tip 1 is electrically connected to the external power source. In the off-state, the electrode tip 1 is electrically disconnected from the external power source.

In the control handle 4 provided in the disclosure, the control mechanism 44 is attached to the housing 41, and the control mechanism 44 includes the control switch 440 and the operation member 441. As such, by manipulating the operation member 441, the control switch 440 can be switched between the on-state and the off-state. In the on-state, the electrode tip 1 is electrically connected to the external power source, so that the external power source can transfer energy to the electrode tip 1, thereby performing ablation puncture or mapping. In the off-state, the electrode tip 1 is electrically disconnected from the external power source, so that energy transferring from the external power source to the electrode tip 1 can be stopped. Therefore, the operator can control the on-state and the off-state of the electric circuit by manipulating the operation member 441, thereby achieving ablation puncture. Compared with an ablation puncture device in the related art where an on-off switch generally requires an assistant to operate a foot switch, which has the disadvantage that the assistant needs to wait for the operator's instructions before stepping on the foot switch, this process takes a certain time, and during this time period, the heartbeat may cause the puncture electrode to deviate from a target position, posing significant potential risks to the patient. By adding the control mechanism to the control handle 4, the operator can directly and immediately operate and control the control mechanism after aligning the position, which is very convenient and facilitates improving the success rate of the puncture.

Exemplarily, in this embodiment, the housing 41 is hollow to define the accommodation space 410. Specifically, the housing 41 includes an upper cover 411 and a lower housing 412. The upper cover 411 and the lower housing 412 cooperatively define the accommodation space 410. The upper cover 411 and the lower housing 412 may be secured together in a manner including, but not limited to, clamping, welding, bonding, and screwing. In this embodiment, the upper cover 411 may be in snap-fit with the lower housing 412r. In order to describe more clearly, the orientations in the disclosure are described based on the axis of the housing 41 that extends horizontally, a direction facing the ground is referred to as downward, and a direction away from the ground is referred to as upward. In FIG. 3, a direction from the upper cover 411 to the lower housing 412 is referred to as upward, and a direction from the lower housing 412 to the upper cover 411 is referred to as downward.

Furthermore, a front rubber sleeve 46 is provided on a distal end of the housing 41. The front rubber sleeve 46 is bonded to the housing 41 by means of glue. The front rubber sleeve 46 is sleeved on an outer periphery of the tube set 2 to reduce the risk of bending of the inner tube, the outer tube, and other structures during an operation, thereby ensuring safety.

In some embodiments, the control handle 4 further includes a power connector 42. The external power source may be electrically connected to the electrode tip 1 via the power connector 42, so that the external power source can deliver energy to the electrode tip 1, thereby puncturing or mapping the target tissue. Specifically, the power connector 42 is disposed at a proximal end of the housing 41 and is configured to be electrically connected to the external power source, where the external power source is used to generate ablation energy such as a radio frequency. The external power source may be, but is not limited to, one or a combination of a radio frequency generator and a pulsed filed generator, or other energy source capable of achieving ablation, which is not limited herein.

In some embodiments, the control handle 4 further includes a fluid supply mechanism 43. The fluid supply mechanism 43 is connected to the middle part of the housing 41. The fluid supply mechanism 43 is in communication with the tube set 2 extending into the accommodation space 410. The fluid supply mechanism 43 includes a fluid supply tubular member 431 and a valve 432. In this embodiment, the fluid supply mechanism 43 is in communication with the tube set 2 through the fluid supply tubular member 431, allowing an injection of fluid during an operation to exhaust air from the tube set 2 and allow a fluid to enter a fluid channel and subsequently enter the human body. In other embodiments, the fluid supply mechanism 43 may be omitted.

One end (i.e., a distal end) of the fluid supply tubular member 431 is connected to the housing 41. Specifically, the housing 41 defines an opening. The opening is in communication with the accommodation space 410. The fluid supply tubular member 431 is located at the opening, extends into the accommodation space 410, and is in sealed connection with the tube set 2, so that the fluid supply tubular member 431 is in communication with the fluid channel. In this embodiment, the fluid supply tubular member 431 extends in the accommodation space towards a distal end of the housing 41, thereby shortening a fluid delivery distance and reducing a fluid flow resistance, and thus enhancing the efficiency of fluid delivery. In some embodiments, part of the fluid supply tubular member 431 in the accommodation space may further extend towards the distal end of the housing 41, and another part of the fluid supply tubular member 431 in the accommodation space may extend towards the proximal end of the housing 41.

Specifically, in this embodiment, the distal end of the fluid supply tubular member 431 in the accommodation space 410 is spaced apart from the distal end of the housing 41, and the proximal end of the tube set 2 extends into the accommodation space 410 and is in communication with the fluid supply tubular member 431, so that the fluid supply tubular member 431 is in communication with the fluid channel. The proximal end of the tube set 2 is bonded to the fluid supply tubular member 431.

The valve 432 is disposed at a proximal end of the fluid supply tubular member 431. In this embodiment, the valve 432 is a three-way valve, including a first port, a second port, and a third port. The first port is in communication with the fluid supply tubular member 431, thereby enabling the valve 431 to be in communication with the tube set 2 and the fluid channel. The second port is configured to be connected to a syringe via a tubular member. The third port is configured to be connected to a negative pressure device via a negative pressure tubular member. In other embodiments, the valve 432 may also be a single-way valve, a two-way valve, or other multi-way valves. It may be noted that, the number of ports of the valve 432 may be designed according to the requirements of the puncture surgery and is not specifically limited in the disclosure. In some embodiments, the valve 432 may be disposed on a fluid supply device, that is, the valve 432 may not be disposed on the fluid supply tubular member 431. For example, the valve 432 is connected to the fluid supply tubular member 431 through a TPU (thermoplastic polyurethane) hose.

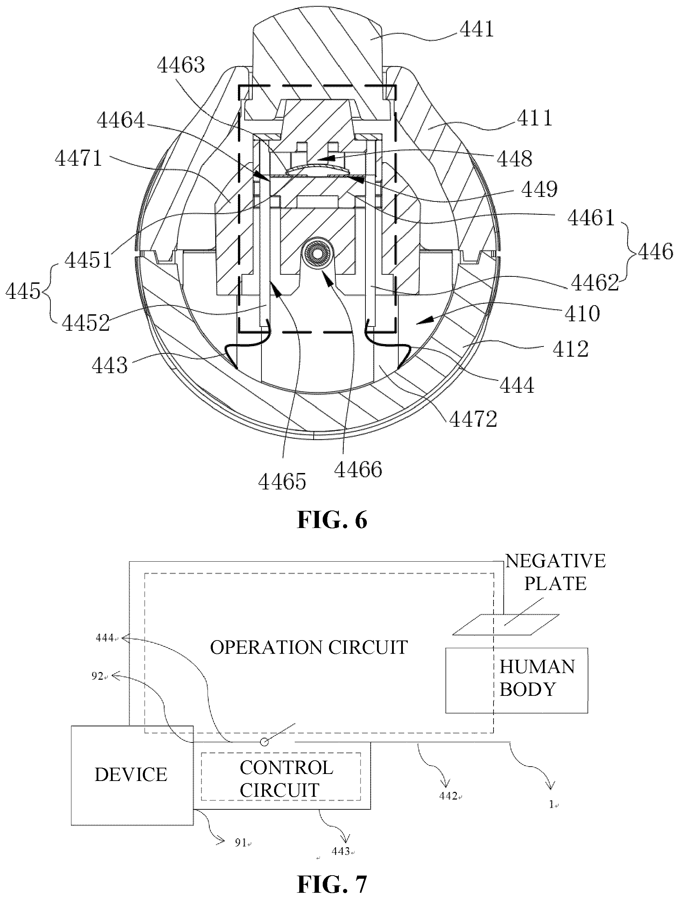

Referring to FIG. 1 and FIG. 6, the control switch 440 includes a first cable 443, a second cable 444, a first connector 445, and a second connector 446. Both the first connector 445 and the second connector 446 are received in the accommodation space 410, and the first connector 445 is spaced from the second connector 446. A distal end of the first cable 443 is electrically connected to the first connector 445, a distal end of the second cable 444 is electrically connected to the second connector 446, and both a proximal end of the first cable 443 and a proximal end of the second cable 444 are electrically connected to the external power source. The first connector 445 is in contact with the second connector 446 when the operation member 441 abuts against the first connector 445 and/or the second connector 446, and the control switch 440 is in the on-state when the first connector 445 is in contact with the second connector 446. When the first connector 445 and/or the second connector 446 are in a released state (i.e., the pressing portion 448 is disengaged from the first connector 445 and/or the second connector 446), the first connector 445 can be isolated from the second connector 446, so that the control switch 440 is in the off-state.

In some embodiments, the operation member 441 is movable relative to the housing 41. The operation member 441 includes a pressing portion 448 for abutting against the first connector 445 and/or the second connector 446. The operation member 441 is configured to drive, under the action of an external force, the pressing portion 448 to abut against the first connector 445 and/or the second connector 446. The operation member 441 is movable under the action of an external force, so that the control switch 440 can realize circuit conduction, thereby enabling the electrode tip 1 to be in electrical connection with the external power source. That is, the electrical connection/disconnection between the electrode tip 1 and the power connector 42 can be controlled by the action of the external force applied on the operation member 441, and further the electrical connection/disconnection between the puncture system and the external power source can be controlled, so that an operator can operate manually, which is very convenient.

In some other embodiments, the operation member 441 is fixed relative to the housing 41. Specifically, the control mechanism 44 further includes a sensor disposed on the operation member 441 and a drive member electrically connected to the sensor. The sensor is configured to generate a touch control signal in response to a preset touch control operation performed on the operation member 441, and the driving member is configured to drive the pressing portion 448 to abut against the first connector 445 and/or the second connector 446 according to the touch control signal, thereby more intelligently controlling the electrical connection/disconnection between the electrode tip 1 and the external power source. It may be understood that, the sensor may be integrally formed with the operation member 441. Alternatively, the sensor may be disposed independent of the operation member 441. The sensor may be, but is not limited to, a pressure sensor, a fingerprint sensor, a distance sensor, an optical coupling sensor, and the like.

Exemplarily, in this embodiment, the operation member 441 is movable relative to the housing 41 in a radial direction of the housing 41. That is, the operation member 441 is movable in the accommodation space 410 under an action of an external force, so that the first connector 445 can be in contact with the second connector 446 to realize circuit conduction. Specifically, the operation member 441 can move up/down in the radial direction of the housing 41. That is, when the operator manipulates the operation member 441, the operator may press or release the operation member 441 by hand, thereby achieving circuit conduction or breaking circuit conduction.

The operation member 441 is exposed to the housing 41, so that it is convenient for a user to manipulate the operation member 441. Exemplarily, in this embodiment, at least part of the operation member 441 extends out of the housing 41. Specifically, the upper cover 411 of the housing 41 defines an opening, part of the operation member 441 is received in the accommodation space 410, and another part of the operation member 441 extends out of the accommodation space 410 through the opening of the upper cover 411. In other embodiments, the entire operation member 441 may be received in the accommodation space 410. For example, an outer periphery of the operation member 441 is slightly lower than or flush with an outer periphery of the housing 41, thereby further preventing a misoperation of pressing of the operation member 441.

In some embodiments, a protective cover may be disposed at a position of the housing 41 corresponding to the operation member 441 to protect the operation member 441. The protective cover is connected to the housing 41, and can be opened/closed relative to the operation member 441. When the protective cover is closed, the operation member 441 is received in a protective space defined by the protective cover and the housing 41, preventing manipulation of the operation member 441 and thus avoiding misoperation. When the protective cover is opened, manipulation of the operation member 441 is permitted.

Optionally, the protective cover is rotatably connected to the housing 41. For example, the protective cover may, but is not limited to, be hinged or pivotally connected between side walls of the opening to achieve opening and closing. The protective cover and the housing 41 may also be detachably connected together in a manner such as snap-fitting or screwing, which is not specifically limited in the disclosure.

The control mechanism 44 further includes a conductive member 442. A distal end of the conductive member 442 extends out of the housing 41 and is electrically connected to the electrode tip 1. A proximal end of the conductive member 442 is electrically connected to the control switch 440 or the external power source. The proximal end of the conductive member 442 may be electrically connected to the control switch 440 or the external power source through the first connector 445 or the second connector 446. Alternatively, the proximal end of the conductive member 442 may be electrically connected to the control switch 440 or the external power source through the first cable 443 or the second cable 444. Alternatively, the proximal end of the conductive member 442 can be directly electrically coupled to the external power source. Exemplarily, in this embodiment, the conductive member 442 is disposed between the control switch 440 and the power connector 42. Specifically, the distal end of the conductive member 442 is electrically connected to the power connector 42, and the proximal end of the conductive member 442 is connected to the first connector 445. Optionally, the conductive member 442 is located within the tube set 2 and the fluid supply tubular member 431.

Exemplarily, in this embodiment, the conductive member 442 is configured as a conducting wire. A distal end of the conducting wire is fixedly connected to the electrode tip 1. The conductive wire may include multiple conductive segments. It may be understood that, to facilitate a better understanding of the conductive member 442 by those skilled in the art, the conductive member 442 is described in detail by using the conductive wire as an example. It may be noted that, the use of a conductive wire for the conductive member 442 is for illustrative purposes only and is not specifically limited in the disclosure. For example, the product type of the conductive member 442 may also be set according to actual requirements. For example, in other embodiments, the conductive member 442 is configured as a conductive tube. The conductive tube may be integrally formed with the electrode tip 1. Alternatively, the conductive tube may be sleeved on the electrode tip 1. Alternatively, the electrode tip 1 may be fixedly connected to a distal surface of the conductive tube.

It may be noted that, an outer periphery of the rest of the conductive member 442, except for end portions used for electrical connection with the first connector 445 and the electrode tip 1, is covered with an insulating material. In other words, two end portions of the conductive member 442 are configured for electrical connections, while the rest of the conductive member 442 is covered with an insulating material.

In this embodiment, there are one first cable 443 and one second cable 444. It may be noted that, the number of the first cable 443 and the number of the second cable 444 are only used for illustrative purposes, and the number of the first cable 443 and the number of the second cable 444 may be set according to actual requirements. For example, in some embodiments, the number of first cables 443 and second cables 444 may also be plural.

The first connector 445 and the second connector 446 are received in the accommodation space 410 and spaced from each other. In this embodiment, both the first connector 445 and the second connector 446 are located at the distal end of the fluid supply tubular member 431 and are spaced apart from the fluid supply tubular member 431. That is, the tube set 2 extends axially, through a position where the first connector 445 is located and a position where the second connector 446 is located, in a direction from the distal end of the housing 41 to the proximal end of the housing 41, and continues to extend towards the proximal end of the housing to be in communication with the fluid supply tubular member 431. In other words, within the accommodation space 410 of the housing 41, the first connector 445 and the second connector 446 do not interfere with the fluid channel of the puncture system.

Alternatively, in some embodiments, the first connector 445 and/or the second connector 446 include a triggering portion 449 corresponding to the pressing portion 448. The pressing portion 448 is configured to abut against the triggering portion 449 to enable the triggering portion 449 of the first connector 445 to be in contact with the second connector 446. In other embodiments, the pressing portion 448 is configured to abut against the triggering portion 449 to enable the triggering portion 449 of the second connector 446 to be in contact with the first connector 445. In other embodiments, the pressing portion 448 is configured to abut against the triggering portion 449 to enable the triggering portion 449 of the first connector 445 to be in contact with the triggering portion 449 of the second connector 446.

Specifically, referring to FIG. 6, the first connector 445 includes a connection piece 4451 and a first pin 4452 spaced from the connection piece 4451. The second connector 446 includes a base 4461 and a second pin 4462. The base 4461 is disposed corresponding to the first connector 445 and located at one side of the connection piece 4451 away from the operation member 441. The first pin 4452 and the second pin 4462 are arranged at intervals on the base 4461, the first pin 4452 is electrically connected to the distal end of the first cable 443, and the second pin 4462 is electrically connected to the distal end of the second cable 444. Two contact points are disposed on the base 4461 at intervals, where one of the two contact points 4463 is electrically connected to the first pin 4452 and the other of the two contact points is electrically connected to the second pin 4462. The triggering portion 449 is the connection piece 4451. The operation member 441 is configured to drive, under the action of an external force, the connection piece 4451 to be in contact the two contact points 4463, to enable the control switch 440 to be in the on-state. Specifically, in this embodiment, the base 4461 includes a first contact and a second contact arranged at intervals, where the first contact is electrically connected to the first pin 4452, and the second contact is electrically connected to the second pin 4462.

Alternatively, in some embodiments, the connection piece 4451 is resilient to ensure that the connection piece 4451 is in full contact with the contact point 4463 after deformation, thereby ensuring good electrical contact between the connection piece 4451 and the two contact points 4463 when the connection piece 4451 is subject to a pressure. In other embodiments, the connection piece 4451 may be rigid. In this embodiment, the pressing portion 448 of the operation member 441 is connected to the connection piece 4451. Optionally, the pressing portion 448 may be fixedly connected to the middle of the connection piece 4451, ensuring that balanced force on both ends of the connection piece 4451, and thus improving the stability and reliability of the connection between the connection piece 4451 and the two contact points 4463. In some embodiments, the pressing portion 448 may also be fixedly connected to the connection piece 4451 at other positions other than the middle of the connection piece 4451, which is not specifically limited in the disclosure. When the operation member 441 abuts against the connection piece 4451 under the action of an external force, the connection piece 4451 moves towards the base 4461, with both ends of the connection piece 4451 respectively abutting against the contact points 4463 of the base 4461.

In some embodiments, the connection piece 4451 includes a connection piece body connected to the operation member 441 and extension portions provided at both ends of the connection piece body in a lengthwise direction of the housing 41. The extension portion extends in a direction parallel to a plane where the contact points 4463 are located, thereby ensuring a contact area between the connection piece 4451 and the two contact points 4463, and thus improving the reliability and stability of electrical contact.

The connection piece 4451 is configured to have a sheet-like structure, thereby simplifying the structure of the connection piece 4451, reducing weight, and saving space.

Exemplarily, in this embodiment, the connection piece 4451 is arc-shaped. Specifically, the connection piece 4451 is bent towards the operation member 441. That is, a convex surface of the connection piece 4451 faces the operation member 441. The connection piece 451 bends and extends in an axial direction of the housing 41. In some embodiments, the connection piece 451 may also be in other shapes, such as having a raised middle portion with flat sides, for example, in an Ω shape or a "JL" shape, which is not specifically limited in the disclosure.

The contact point 4463 protrudes outwards from the base 4461 towards the connection piece 4451. The first pin 4452 is electrically connected to the first cable 443. The second pin 4462 is electrically connected to the second cable 444. When the connection piece 4451 is in contact with the two contact points 4463, the first pin 4452 is electrically connected to the second pin 4462, and the control switch 440 is in the on-state. When the connection piece 4451 is spaced apart from the two contact points 4463, the first pin 4452 is disconnected from the second pin 4462, and the control switch 440 is in the off-state. In this embodiment, the proximal end of the conductive member 442 is electrically connected to the first cable 443, and in turn is electrically connected to the first pin 4452, thereby achieving electrical connection between the conductive member 442 and the first connector 445.

When the operation member 441 is pressed (for example, an external force is applied to the operation member 441), the connection piece 4451 moves towards the base 4461 until the connection piece 4451 comes in contact with the contact point 4463, thereby achieving electrical connection between the first connector 445 and the second connector 446, and thus enabling the electrode tip 1 to be in electrical connection with the power connector 42. When the operation member 441 is released (for example, the external force applied to the operation member 441 is removed), the connection piece 4451 moves away from the base 4461 until the connection piece 4451 is spaced apart from the contact points 4463. As such, by controlling the position of the connection piece 4451 relative to the contact points 4463, the control switch 440 can be controlled to be in the on-state or the off-state, which is simple in structure and easy to operate.

In some embodiments, the triggering portion 449 is resilient, the triggering portion 449 is deformable under the action of an external force, and is capable of restoring from deformation to reset when the external force is removed. In other embodiments, the control mechanism 44 further includes a reset member. The reset member is disposed between the first connector 445 and the second connector 446, and the reset member is configured to provide a force for resetting the triggering portion 449. In other embodiments, the control mechanism 44 further includes an elastic member configured to provide a force to enable to the operation member 441 to move away from the first connector 445. As such, it is ensured that the first connector 445 and the second connector 446 can be driven to move towards and be in contact with each other to enable the control switch 440 to be in the on-state when the triggering portion 449 is pressed, and that the first connector 445 and the second connector 446 can be driven to move away from and be spaced apart from each other to enable the control switch 440 to be in the off-state when a pressure applied on the triggering portion 449 is remove, thereby facilitating quick resetting of the operation member 441, and accurately controlling the energy output of the external power source, and improving the accuracy of surgery or mapping.

In some embodiments, the control mechanism 44 further includes a fixing member 447. The fixing member 447 is insulated from the first connector 445 and the second connector 446. The base 4461 is disposed in the accommodation space 410 via the fixing member 447. The fixing member 447 includes a fixing seat 4471 and multiple supporting legs 4472 supporting the fixing seat 4471. The multiple supporting legs 4472 are fixed in the housing 41 at intervals, and the fixing seat 4471 defines a groove 4464 for receiving the base 4461 and through-holes 4465 for allowing the first pin 4452 and the second pin 4462 to extend through. The through-holes 4465 are spaced apart from the multiple supporting legs 4472, and both the first pin 4452 and the second pin 4462 extend out of the fixing seat 4471.

In some embodiments, the fixing seat 4471 further defines a channel 4466. The tube set 2 extends through the channel 4466. Specifically, the groove 4464 is defined on a surface of the fixing seat 4471 facing the base 4461, and the channel 4466 is defined a surface of the fixing seat 4471 facing away from the base 4461. Exemplarily, in this embodiment, an axis of the through-hole 4465 extends in the radial direction of the housing 41. In some embodiments, the axis of the through-hole 4465 may be angled relative to the radial direction of the housing 41 at a preset angle, which is not specifically limited in the disclosure.

The second connector 446 is fixed in the accommodation space 410 of the housing 41 through the fixing member 447. The connection piece 4451 is capable of moving towards or away from a circuit board of the second connector 446, thereby achieving electrical connection through contact or achieving electrical disconnection through non-contact.

A circuit of the control mechanism may operate as follows. When the operation member 441 is pressed, and the circuit is turned on to transmit a signal to an operation circuit, so that the operation circuit is turned on, and then radio frequency energy output by the external power source can be transmitted to the electrode tip 1, thereby realizing puncturing. Specifically, referring to FIGS. 1, 6, and 7, the circuit principle of the control mechanism 44 is as follows.

The first cable 443 and the second cable 444 are connected to two plugs of a connection line of the external power source via the power connector 42. In the foregoing manner, regardless of which plug the energy passes through, it can ensure that the energy reaches the electrode tip 1. Specifically, the first cable 443 corresponds to the first seat 91, and the second cable 444 corresponds to the second seat 92. Assuming that energy enters through the first seat 91, a connection between the first cable 443 and the first seat 91 and a connection between the second cable 444 and the second seat 92 are both positive, and in this case, the energy may flow along the first cable 443 through a welding point between the first cable 443 and the conductive member 442, thereby turning on the operation circuit. When it is reversely connected, that is, the second cable 444 is connected to the first seat 91, the energy may flow along the second cable 444 through the welding point between the first cable 443 and the conductive member 442 to the conductive member 442, thereby turning on the operation circuit.

The electrode tip 1 is configured to be in contact with the human body. The first cable 443 and the second cable 444 are connected to two plugs of a radio frequency energy connection line via the radio frequency power connector 42. In this way, regardless of which plug the radio frequency energy passes through, it can ensure that the operation circuit can be turned on, thereby delivering the radio frequency energy to the electrode tip 1 to achieve puncturing.

It may be noted that, after the operation member is pressed down, the first cable 443 and the second cable 444 form a control loop, a device receives a first control signal that enables the device to generate a second control signal, and the second control signal triggers turning-on of the operation circuit, controlling the energy delivery to the electrode tip 1.

In the control handle 4 in this embodiment, the control mechanism is provided, allowing the operator to conveniently control the connection and disconnection of a circuit by pressing or releasing the operation member 441. In addition, a foot switch may also be pressed during surgery to achieve circuit connection.

### Second Embodiment

Referring to FIGS. 1, 8, 9, and 10 together, in the second embodiment of the disclosure, the difference between a control handle 5 and the control handle 4 in the first embodiment lies in the specific structures of the first connector 545 and the second connector 546.

The fluid supply tubular member 531 extends towards a distal end of a housing 51. The first connector 545 and the second connector 546 are both received in the fluid supply tubular member 531. In other embodiments, the first connector 545 and the second connector 546 may be located outside the fluid supply tubular member 531, or there may be no fluid supply mechanism 53.

Specifically, the fluid supply tubular member 531 extends within the housing 51 towards the distal end of the housing 51. Both a proximal end of the fluid supply tubular member 531 and the valve 532 of the fluid supply mechanism 53 are the same as those in the first embodiment. For the specific structure, reference may be made to the detailed illustration of the first embodiment, which is not repeated herein.

Part of the fluid supply tubular member 531 which is received in the housing 51 also extends towards a proximal end of the housing 51. The fluid supply tubular member 531 is further provided with a tail end for blocking at the proximal end of the housing 51. The tail end blocks off the fluid supply tubular member 531.

The fluid supply tubular member 531 defines at a position corresponding to the operation member 541 and an opening for allowing the operation member 541 to extend through, and the operation member 541 is in an interference fit with the opening of the fluid supply tubular member 531.

The first connector 545 is in a sheet shape and extends in an axial direction of the housing 51. The first connector 545 has a free portion 5454, and the free portion 5454 is located corresponding to the opening of the fluid supply tubular member 531.

The triggering portion 449 includes the free portion 5454 that is resilient. The free portion 5454 is disposed on the first connector 545, and a free end of the free portion 5454 is movable within the accommodation space 410. The second connector 546 is located at one side of the first connector 545 away from the operation member 541. The free portion 5454 is capable of driving, under an action of an external force, the free end of the free portion 5454 to move towards and be in contact with the second connector 546, to enable the control switch 440 to be in an on-state.

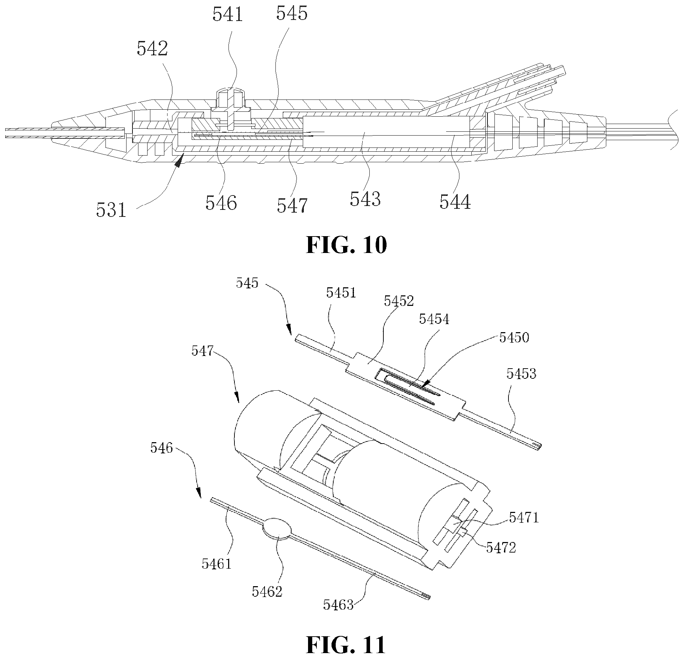

Specifically, referring to FIG. 11, the first connector 545 includes a distal connection portion 5451, a middle portion 5452, and a proximal connection portion 5453 which are sequentially arranged in a direction from a distal end of the first connector 545 to a proximal end of the first connector 545. Each of the distal connection portion 5451 and the proximal connection portion 5453 has a width smaller than that of the middle portion 5452. In this embodiment, the distal connection portion 5451 and the proximal connection portion 5453 are elongated.

The first connector 545 defines a through-hole 5450 at the middle portion 5452 of the first connector 545, thereby forming the free portion 5454. The free portion 5454 extends in an axial direction of the housing 51. Exemplarily, in this embodiment, the through-hole 5450 is an arc-shaped hole. The free portion 5454 is formed corresponding to a concave surface of the arc-shaped hole. That is, a portion encircled in the arc-shaped hole forms the free portion 5454. In this embodiment, specifically, the through-hole 5450 is a U-shaped hole, so that the free portion 5454 is in an elongated shape. The free portion 5454 forms the free end corresponding to a concave surface of the arc-shaped hole, and the free end is movable in a radial direction of the housing 51. In some embodiments, the through-hole 5450 may also be, but is not limited to, a L-shaped hole, a C-shaped hole, etc.

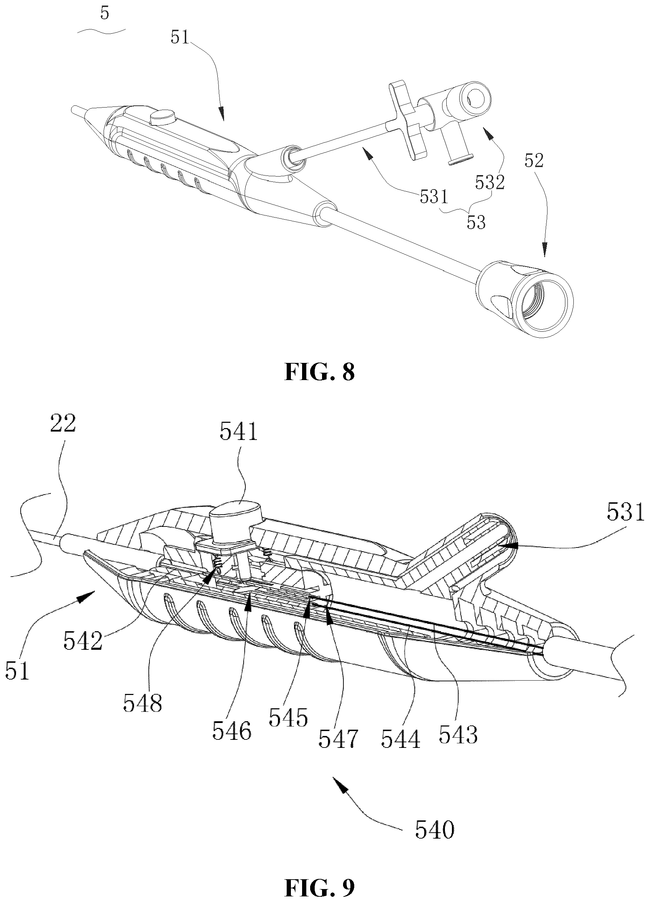

Referring to FIGS. 8 to 11 again, the distal end of the first connector 545 is electrically connected to a proximal end of the conductive member 542, and the proximal end of the first connector 545 is electrically connected to a first cable 543. In this embodiment, the distal end of the first connector 545 is welded and connected to the proximal end of the conductive member 542, and the proximal end of the first connector 545 is welded and connected to the first cable 543. Specifically, a distal end of the distal connection portion 5451 is electrically connected to the conductive member 542, and a proximal end of the proximal connection portion 5453 is electrically connected to the first cable 543.

Exemplarily, in this embodiment, the second connector 546 is spaced apart from the first connector 545. That is, the second connector 546 also extends in the axial direction of the housing 51. Optionally, the second connector 546 is parallel to and spaced apart from the first connector 545, thereby saving space. In some embodiments, an extending direction of the second connector 546 may be different from an extending direction of the first connector 545. Specifically, the second connector 546 includes a distal connection rod 5461, a contact portion 5462, and a proximal connection rod 5463 arranged in a direction from a distal end of the second connector 546 to a proximal end of the second connector 546. The contact portion 5462 is disposed corresponding to the free portion 5454. The contact portion 5462 is circle-shaped. The distal connection rod 5461 and the proximal connection rod 5463 are rod-shaped. In some embodiments, the shape of the contact portion 5462 may be, but is not limited to, in an oval shape, a square shape, a polygonal shape, an irregular shape, or the like, which is not specifically limited in the disclosure.

The proximal end of the second connector 546 is electrically connected to the second cable 544. In this embodiment, the proximal end of the second connector 546 is welded and connected to the second cable 544. Specifically, a proximal end of the proximal connection rod 5463 is electrically connected to second cable 544.

The control mechanism 540 further includes a protective film (not illustrated) that is insulating and waterproof. The protective film covers an outer periphery of the first connector 545 and an outer periphery of the second connector 546. Specifically, the protective film covers the first connector 545 and the second connector 546 to isolate the first connector 545 and the second connector 546 from fluid carried within the fluid supply tubular member 531.

The protective film defines three connection holes. The conductive member 542, the first cable 543, and the second cable 544 respectively extend through the three connection holes of the protective film to be electrically connected to the first connector 545 and the second connector 546. The conductive member 542, the first cable 543, and the second cable 544 are in sealed connection with the protective film at the connection holes. The control mechanism 540 also includes a fixing member 547 for supporting and securing the first connector 545 and the second connector 546. The fixing member 447 is insulated from the first connector 445 and the second connector 446.

An axis of the fixing member 547 extends in the axial direction of the housing 51. In this embodiment, the fixing member 547 is substantially cuboidal, and part of the fixing member 547 close to an outer periphery of the operation member 541 is arc-shaped to match an inner periphery of the fluid supply tubular member 531.

In some embodiments, the fixing member 547 is fixedly connected to the fluid supply tubular member 531. Specifically, two protruding bars are provided at intervals on an outer periphery of the fixing member 547 and extend in the axial direction of the housing 51. An inner circumferential wall of the fluid supply tubular member 531 defines two clamping grooves, and the two protruding bars are engaged with the two clamping grooves in one-to-one correspondence, so that the fixing member 547 is fixed to the fluid supply tubular member 531.

The fixing member 547 has a through-hole 5471 and an accommodation groove 5472. The through-hole 5471 extends through both ends of the fixing member 547 that are arranged along the axis of the fixing member 547. The accommodation groove 5472 has an opening facing the proximal end of the housing 51. The first connector 545 extends through the through-hole 5471. That is, both ends of the first connector 545 extend out of the fixing member 547. The second connector 546 is disposed in the accommodation groove 5472. That is, the distal end of the second connector 546 is received in the fixing member 547, and the proximal end of the second connector 546 extends out of the fixing member 547.

The through-hole 5471 is in communication with the accommodation groove 5472 at a position corresponding to the free portion 5454 of the first connector 545, so that the free portion 5454 can be in contact with the contact portion 5462 of the second connector 546.

When the operation member 541 is pressed, the operation member 541 will move in a radial direction of the housing 51 to drive the free portion 5454 to approach the contact portion 5462 until the free portion 5454 is in contact with the contact portion 5462, thereby achieving circuit conduction.

The control mechanism 540 further includes an elastic member 548 to provide a force to enable the operation member 541 to move away from the first connector 545. Exemplarily, in this embodiment, the elastic member 548 is implemented as two elastic members 548. The two elastic members 548 are arranged at intervals in a circumferential direction of the housing 51. It may be noted that, the number of the elastic members 548 is merely used for illustration, which is not specifically limited in the disclosure. The number of the elastic members 548 may be set according to actual requirements. For example, the number of the elastic members 548 may also be one, three, or more than three. In this embodiment, two ends of each elastic member 548 abut against the operation member 541 and an outer wall of the fluid supply tubular member 531 at the opening of the fluid supply tubular member 531, respectively. The fluid supply tubular member 531 has two lower hook rings on the outer wall of the fluid supply tubular member 531 at the opening of the fluid supply tubular member 531. The two elastic members 548 are respectively connected to the two lower hook rings in one-to-one correspondence. The operation member 541 has two upper hooks on a surface of the operation member 541 facing the fluid supply tubular member 531. The two elastic members 548 are respectively connected to the two upper hooks in one-to-one correspondence. In other embodiments, two guide posts may be provided on the fixing member 547 or the fluid supply tubular member 531 at positions corresponding to the operation member 541, so that the two elastic members 548 can be sleeved on the two guide posts in one-to-one correspondence. The elastic member 548 may be configured as, but not limited to, an elastic structure such as a spring or a dome.

In this embodiment, the operation member 541 is configured to move in the radial direction of the housing 51 to drive the free portion 5454 of the first connector 545 to move in the radial direction of the housing 51 to be in contact with the contact portion 5462 of the second connector 546, thereby achieving circuit conduction.

For other features such as the power connector 52 of the control handle 5 in this embodiment, reference may be made to the first embodiment, and details are not described herein again.

### Third Embodiment

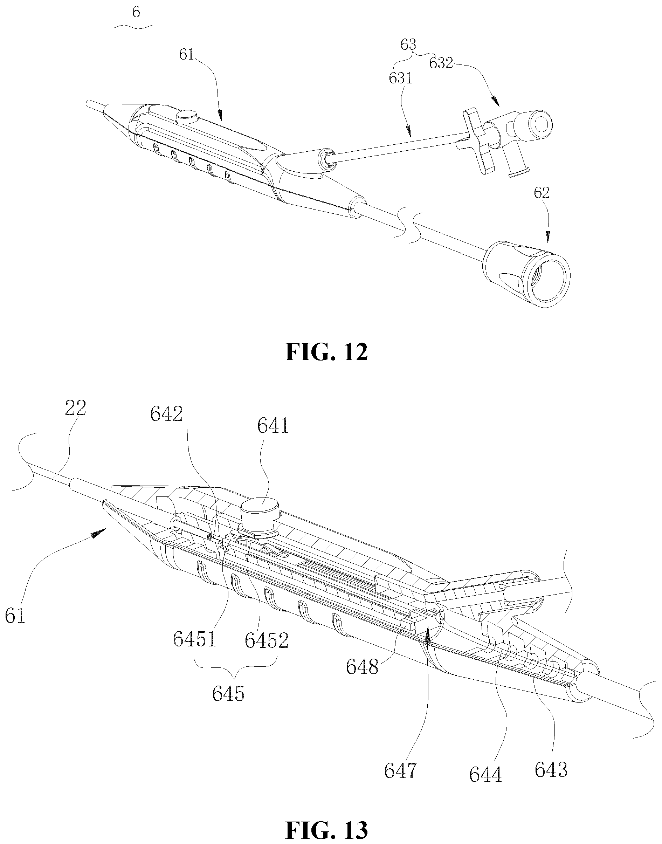

Referring to FIGS. 1, 12, 13, and 14 together, in the third embodiment of the disclosure, the difference between a control handle 6 and the control handle 5 in the second embodiment lies in the specific structures of a first connector 645 and a second connector 646.

The fluid supply tubular member 631 and the valve 632 of the fluid supply mechanism 63, and the power connector 62 are the same as those in the second embodiment, and reference may be made to the illustration of the second embodiment, which is not repeated herein. In other embodiments, the first connector 645 and the second connector 646 may be located outside the fluid supply tubular member 631, or there may be no fluid supply mechanism 63.

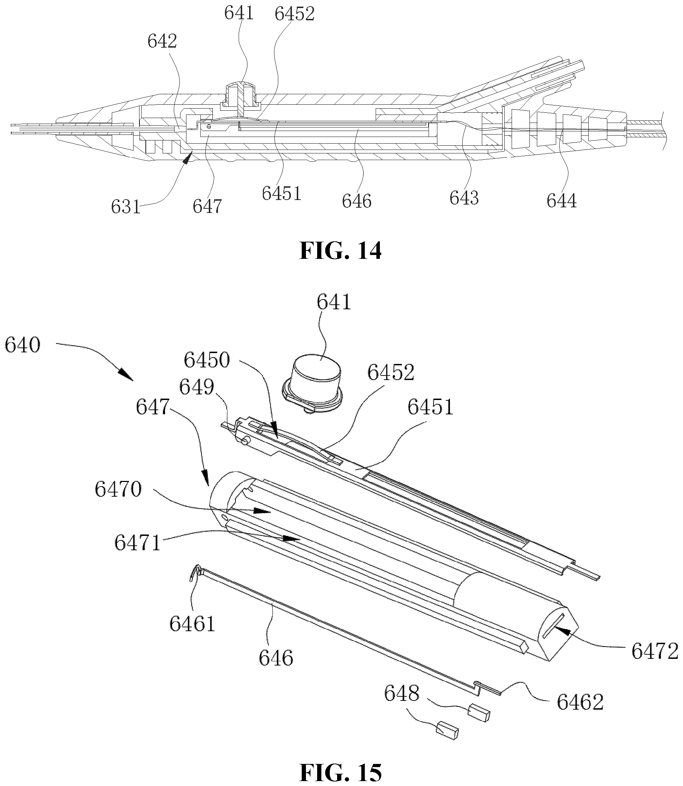

Referring to FIG. 15, the first connector 645 includes a resilient piece 6451 and a conductive piece 6452. The resilient piece 6451 is electrically connected to the conductive piece 6452. Two ends of the conductive piece 6452 are both fixed to the resilient piece 6451, and the resilient piece 6451 is electrically connected to a first cable 643.

Specifically, a proximal end of the resilient piece 6451 is electrically connected to the first cable 643, and a distal end of the resilient piece 6451 is electrically connected to a conductive member 642. In this embodiment, the proximal end of the resilient piece 6451 is welded and connected to the first cable 643, and the distal end of the resilient piece 6451 is welded and connected to the conductive member 642. In other embodiments, the resilient piece 6451 may be connected to the first cable 643 and the conductive member 642 through a mounting structure, or through snap-fitting.

The resilient piece 6451 defines a through-hole 6450 corresponding to the operation member 641.

The conductive piece 6452 is disposed corresponding to the through-hole 6450 and located between the resilient piece 6451 and the operation member 641. In this embodiment, the conductive piece 6452 serves as the triggering portion. A distal end of the conductive piece 6452 is fixed to the distal end of the resilient piece 6451, and the conductive piece 6452 is electrically connected to the resilient piece 6451. Specifically, the distal end of the conductive piece 6452 is welded and connected to the distal end of the resilient piece 6451, thereby achieving mechanical connection and electrical connection.

In some embodiments, the conductive piece 6452 is in an arc-shape. A convex surface of the conductive piece 6452 faces the operation member 641. A periphery of the conductive piece 6452 extends in the axial direction of the housing 61.

The second connector 646 is disposed corresponding to the through-hole 6450, and is electrically connected to the second cable 444. The second connector 446 is located at one side of the resilient piece 6451 away from the operation member 441. Specifically, the second connector 646 extends to a proximal end of the housing 61 and is electrically connected to the second cable 444. In this embodiment, the second connector 646 extends in the axial direction of the housing 61.

The second connector 646 has a protruding portion 6461 electrically connected to the conductive piece 6452. The protruding portion 6461 extends in a circumferential direction of the housing 61. In this embodiment, the second connector 646 is provided at the distal end thereof with the protruding portion 6461 facing the conductive piece 6452. The protruding portion 6461 is in an arc-shape. A convex surface of the protruding portion 6461 faces a concave surface of the conductive piece 6452.

The operation member 641 is configured to drive, under the action of an external force, a middle portion of the conductive piece 6452 to deform to be in contact with the second connector 646, to enable the control switch 440 to be in the on-state. When the external force is removed, the operation member 641 allows the middle portion of the conductive piece 6452 to restore from deformation to be spaced apart from the second connector 646, to enable the control switch 440 to be in the off-state. Specifically, the operation member 641 is configured to drive, under the action of an external force, the conductive piece 6452 to move towards the second connector 646 to be in contact with the second connector 646, thereby achieving circuit conduction. Exemplarily, in this embodiment, when the operation member 641 moves in the radial direction of the housing 41, the conductive piece 6452 can be brought into contact with the convex surface of the protruding portion 6461.

Furthermore, the control mechanism 640 further includes a fixing member 647. The fixing member 647 is fixed in the fluid supply tubular member 631 and extends in the axial direction of the housing 61.

Referring to FIG. 15 again, the fixing member 647 is hollow to define an accommodation space 6470 for receiving the first connector 645 and the second connector 646. The fixing member 647 defines an opening 6471 on a top of the fixing member 647. The opening 6471 of the fixing member 647 is in communication with the accommodation space 6470 for allowing the operation member 641 to be in contact with the conductive piece 6452.

In this embodiment, a connection between the fixing member 647 and the fluid supply tubular member 631 may refer to the second embodiment. In other words, the fixing member 647 may be connected to the fluid supply tubular member 631 through engagement between the protruding bars and the clamping grooves. Furthermore, a stopper 648 is further provided between the fluid supply tubular member and the fixing member 647. The stopper 648 is disposed at a proximal end of the protruding bar of the fixing member 647, and the stopper 648 is in interference fit with the clamping groove of the fluid supply tubular member, thereby avoiding the fixing member 647 from moving towards the proximal end of the housing 61.

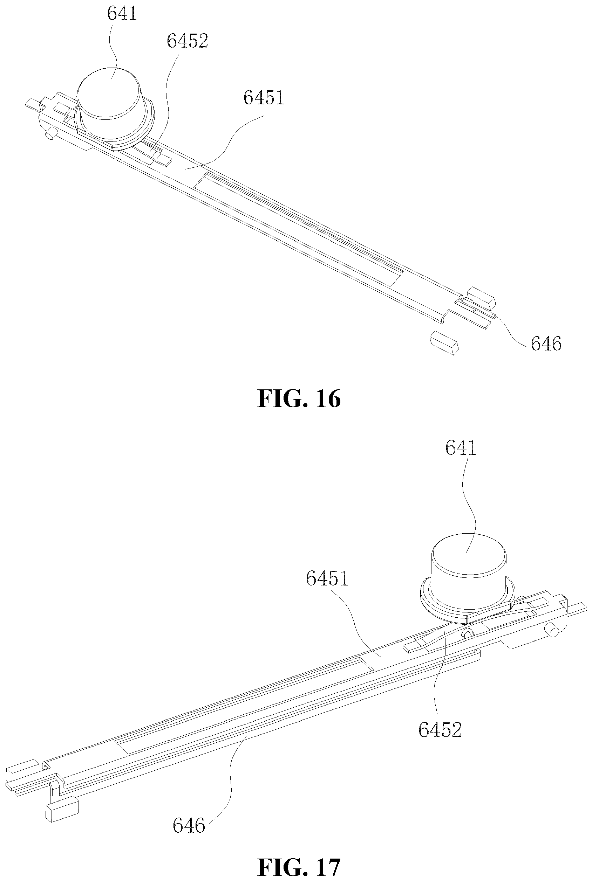

Referring to FIG. 16 and FIG. 17, both the distal end and the proximal end of the resilient piece 6451 are connected to the fixing member 647. In this embodiment, the distal end of the resilient piece is connected to the fixing member 447 via a fixing shaft 649. Specifically, the fixing member 647 defines a fixing hole on each of both side faces of a distal end of the fixing member 647, the resilient piece 6451 has a mounting plate on each of both side faces of the distal end of the resilient piece 6451, and the mounting plate defines a mounting hole. The resilient piece 6451 is received in the accommodation space 6470 of the fixing member 647. The fixing shaft 649 extends through both the fixing hole of the fixing member 647 and the mounting hole of the mounting plate, so that the fixing member 647 is connected to the distal end of the resilient piece 6451. In some embodiments, the fixing shaft 649 can be integrally formed with the resilient piece 6451. That is, the fixing shaft 649 resiliently protrudes from the resilient piece 6451. In some other embodiments, the resilient piece 6451 may be connected to the fixing member 647 via a mounting structure such as a snap-fit or a screw.

The fixing member 647 defines a through-hole 6472 at the proximal end of the fixing member 647. The proximal end of the first connector 645 and a proximal end of the second connector 646 extend through the through-hole 6472. Specifically, the proximal end of the first connector 645 extends out of the fixing member 647 through the through-hole 6472. That is, the distal end of the resilient piece 6451 is connected to the fixing member 647 through the fixing shaft 649, and the proximal end of the resilient piece 6451 is placed in the through-hole 6472, thereby achieving the connection between the fixing member 647 and the resilient piece 6451.

The proximal end of the second connector 646 has a bending portion. The bending portion 6462 is bent towards the resilient piece 6451, so that the proximal end of the resilient piece 6451 and the proximal end of the second connector 646 can be located at the same radial height. The proximal end of the second connector 646 extends out of the proximal end of the fixing member 647 through the through-hole 6472, that is, the proximal end of the second connector 646 is placed in the through-hole 6472, so that the fixing member 647 is connected to the second connector 646.

The conductive piece 6452 can contact the operation member 641 through the opening 6471 of the fixing member 647 so as to move along with the movement of the operation member 641.

In the third embodiment, the other features of the control handle 6 can refer to the second embodiment, and will not be described in detail herein.

### Fourth Embodiment

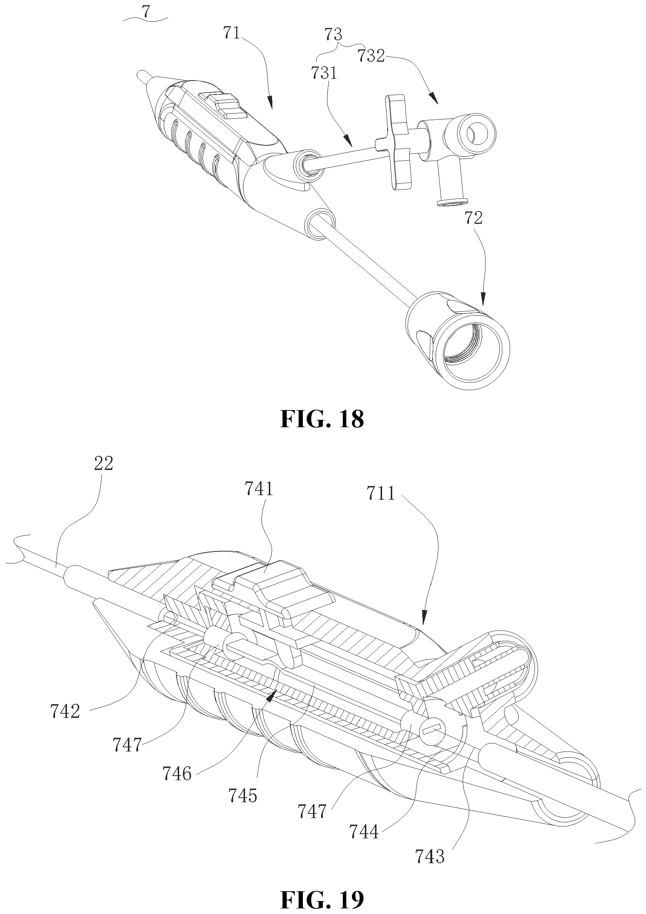

Referring to FIGS. 1, 18, 19, and 20, in the fourth embodiment of the disclosure, the difference between a control handle 7 and the control handle 5 in the second embodiment lies in the specific structure of a first connector 745 and a second connector 746, and a moving direction of the operation member 741.

The fluid supply tubular member 731 and the valve 732 of the fluid supply mechanism 73, and the power connector 72 are the same as those in the second embodiment, and reference may be made to the illustration of the second embodiment, which is not repeated herein. In other embodiments, the first connector 745 and the second connector 746 may be located outside the fluid supply tubular member 731, or there may be no fluid supply mechanism 73.

In this embodiment, the operation member 741 is movable in an axial direction of the housing 71. Specifically, the housing 71 defines an opening 711 extending in the axial direction. The operation member 741 is slidable along the opening 711. The opening 711 is defined on an upper cover of the housing 71 and extends in the axial direction, so that the operation member 741 can move in the axial direction.

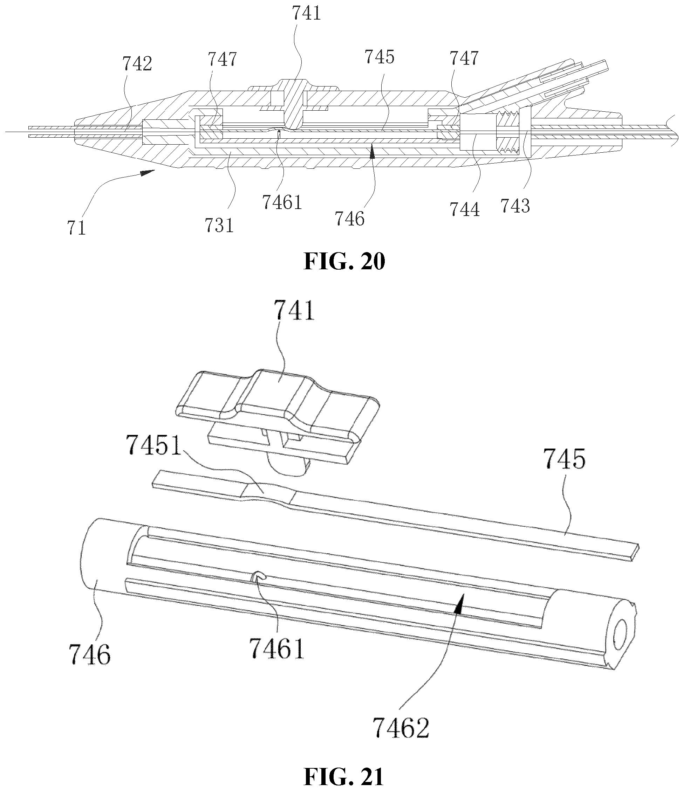

Referring to FIG. 21, in some embodiments, a surface of the operation member 741 close to the first connector 745 is arc-shaped, and the arc-shape periphery extends in the axial direction of the housing 71, so that the operation member 741 can move smoothly in the axial direction.

The first connector 745 is in a sheet shape and extends in the axial direction of the housing 71. The proximal end of the first connector 745 is electrically connected to the first cable 743, and the distal end of the first connector 745 is electrically connected to the conductive member 742.

The first connector 745 has a first protrusion 7451 protruding toward the operation member 741. Specifically, the first protrusion 7451 is arc-shaped, a convex surface of the first protrusion 7451 faces the operation member 741, and the first protrusion 7451 extends in the axial direction of the housing 71.

A proximal end of the second connector 746 is electrically connected to the second cable 744.

The second connector 746 has a second protrusion 7461 corresponding to the first protrusion 7451. The second protrusion 7461 is located on one side of the second connector 746 facing the first connector 745, that is, the second protrusion 7461 and the operation member 741 are arranged at two sides of the first protrusion 7451, respectively. The second protrusion 7461 extends in a circumferential direction of the housing 71. The triggering portion includes the first protrusion 7451 and the second protrusion 7461. The first protrusion 7451 is disposed on a path along which the operation member 741 is movable. When the operation member 741 moves to a position where the first protrusion 7451 is located, a pressing portion of the operation member 741 abuts against the first protrusion 7451, so that the first protrusion 7451 is deformed to be in contact with the second protrusion 7461 to enable the control switch 440 to be in the on-state. When the operation member 741 moves to a position away from the first protrusion 7451, the pressing portion of the operation member 741 is spaced apart from the first protrusion 7451, so that the first protrusion 7451 can restore from deformation to be spaced apart from the second protrusion 7461, to enable the control switch 440 to be in the off-state.

The second connector 746 is in a tubular shape, and is in snap-fit with the fluid supply tubular member 731. Exemplarily, the fluid supply tubular member 731 has two protruding bars on an outer periphery of the fluid supply tubular member 731. The two protruding bars extend in the axial direction. An inner wall of the fluid supply tubular member 731 defines two clamping grooves. The two protruding bars are in snap-fit with the two clamping grooves in one-to-one correspondence.

The second connector 746 defines an opening 7462 for allowing the operation member 441 to move. The opening 7462 is in communication with an inner cavity of the second connector 746. Specifically, a peripheral wall of the second connector 746 facing the operation member 741 defines the opening 7462 for allowing the operation member 741 to move. The first protrusion 7451 is disposed corresponding to the opening 7462. That is, the first protrusion 7451 is disposed on a path along which the operation member 741 is movable. When the operation member 741 moves to the position where the first protrusion 7451 is located, the first protrusion 7451 is moved to be in contact with the second protrusion 7461, thereby achieving circuit conduction.

Fitting members 747 are respectively sleeved on two ends of the first connector 745. The second connector 746 is sleeved on an outer periphery of each of the fitting members 747, so that the first connector 745 is received in the inner cavity of the second connector 746, and the first connector 745 is insulated from the second connector 746 via the fitting members 747. The fitting member 747 is made of an insulating material. Alternatively, an insulating layer is disposed on a surface of the fitting member 747. Alternatively, the fitting members 747 have insulating structures disposed at positions corresponding to the first connector 745 and the second connector 746. The fitting members 747 are respectively sleeved on two end of the first connector 745 and received within the second connector 746.

In this embodiment, when the operation member 741 is in a free state, the operation member 741 is located at the proximal end of the first protrusion 7451. In the case where it needs to achieve circuit conduction, the operation member 741 can be pushed to move in a direction from a proximal end of the first protrusion 7451 to a distal end of the first protrusion 7451, thereby gradually approaching the first protrusion 7451. When the operation member 741 reaches a position where the first protrusion 7451 is located, the first protrusion 7451 can be driven to move towards the second protrusion 7461 to enable the first protrusion 7451 to be in contact with the second protrusion 7461, thereby achieving circuit conduction. In the case where it needs to break circuit conduction, the operation member 741 may be pushed to move towards the proximal end of the first protrusion 7451. In other embodiments, when the operation member 741 is in the free state, the operation member 741 may be located at the distal end of the first protrusion 7451.

For other features of the control handle 7 in this embodiment, reference may be made to the second embodiment, and details are not repeatedly described herein.

In the above embodiments of the control handle (including the second embodiment, the third embodiment, and the fourth embodiment), the first connector and the second connector may be directly or indirectly fixed in the accommodation space of the housing via the fixing member, and are located at a proximal end of part of the fluid supply tubular member that is within the housing. The fixing member may be in a tubular shape, and may also be in other shapes according to actual requirements.

In this case, the conductive member extends in the fluid supply tubular member, the distal end of the conductive member is electrically connected to the electrode tip, and the proximal end of the conductive member extends out of the proximal end of the part of the fluid supply tubular member that is within the housing and thus is electrically connected to the first connector.

In some embodiments, the internal structure of the operation member can be designed such that pressing the operation member once will achieve and remain electrical connection between the first connector and the second connector, and that pressing the operation member again will enable the first connector to be spaced apart from the second connector.

It can be seen from the above technical solutions that the disclosure has at least the following advantages and positive effects.

The control handle of the disclosure includes the housing and the control mechanism. The control mechanism includes the operation member and the control switch. By manipulating the operation member, circuit conduction can be achieved via the control switch, thereby achieving an electrical connection between the electrode tip and the external power source. In other words, the operator can control the on-state and the off-state of the electric circuit by manipulating the operation member, thereby achieving ablation puncture. Compared with an ablation puncture device in the related art where an on-off switch generally requires an assistant to operate a foot switch, which has the disadvantage that the assistant needs to wait for the operator's instructions before stepping on the foot switch, this process takes a certain time, and during this time period, the heartbeat may cause the puncture electrode to deviate from a target position, posing significant potential risks to the patient. By adding the control mechanism to the control handle, the operator can directly and immediately operate and control the control mechanism after aligning the position, which is very convenient and facilitates improving the success rate of the puncture.

It may be noted that the specific technical solutions in the foregoing embodiments can be applied interchangeably.

While the disclosure has been described with reference to various exemplary embodiments, it is to be understood that the terminology used is for descriptive and illustrative purposes only, rather than limiting purposes. Since the disclosure may be embodied in various forms without departing from its spirit or essence, it may be understood that the above embodiments are not limited to any of the foregoing details, but should be broadly construed within the spirit and scope defined by the appended claims. Therefore, all variations and modifications that fall within the scope of the claims or their equivalents are intended to be covered by the appended claims.

## Claims

1. A control handle, comprising:
a housing defining an accommodation space therein; and
a control mechanism comprising:
an operation member attached to the housing; and
a control switch disposed in the accommodation space, wherein the control switch is operable to be electrically connected to an electrode tip and an external power source;
wherein the operation member is configured to trigger the control switch to be in an on-state or an off-state, wherein in the on-state, the electrode tip is electrically connected to the external power source, and in the off-state, the electrode tip is electrically disconnected from the external power source.

2. The control handle of claim 1, wherein the control switch comprises:
a first connector and a second connector, wherein both the first connector and the second connector are received in the accommodation space, and the first connector is spaced apart from the second connector; and
a first cable and a second cable, wherein a distal end of the first cable is electrically connected to the first connector, a distal end of the second cable is electrically connected to the second connector, and both a proximal end of the first cable and a proximal end of the second cable are electrically connected to the external power source;
wherein the first connector is in contact with the second connector when the operation member abuts against the first connector and/or the second connector, and the control switch is in the on-state when the first connector is in contact with the second connector.

3. The control handle of claim 2, wherein the operation member comprises a pressing portion configured to abut against the first connector and/or the second connector, the first connector is in contact with the second connector when the pressing portion abuts against the first connector and/or the second connector, and the control switch is in the on-state when the first connector is in contact with the second connector;
the operation member is movable relative to the housing, and the operation member is configured to drive, under the action of an external force, the pressing portion to abut against the first connector and/or the second connector; or
the operation member is fixed relative to the housing, and the control mechanism further comprises a sensor disposed on the operation member and a drive member electrically connected to the sensor, wherein the sensor is configured to generate a touch control signal in response to a preset touch control operation performed on the operation member, and the drive member is configured to drive the pressing portion to abut against the first connector and/or the second connector according to the touch control signal.

4. The control handle of claim 3, wherein the first connector and/or the second connector comprises a triggering portion corresponding to the pressing portion, and the pressing portion is configured to abut against the triggering portion to enable the triggering portion of the first connector to be in contact with the second connector, or to enable the triggering portion of the second connector to be in contact with the first connector, or to enable the triggering portion of the first connector to be in contact with the triggering portion of the second connector.

5. The control handle of claim 4, wherein the triggering portion is resilient, the triggering portion is deformable under the action of the external force, and is capable of restoring from deformation to reset when the external force is removed; and/or
the control mechanism further comprises a reset member, wherein the reset member is disposed between the first connector and the second connector, and the reset member is configured to provide a force for resetting the triggering portion.

6. The control handle of any one of claims 2 to 5, wherein the control mechanism further comprises an elastic member configured to provide a force to enable the operation member to move away from the first connector.

7. The control handle of claim 4, wherein the control mechanism further comprises a conductive member, wherein a distal end of the conductive member extends out of the housing and is electrically connected to the electrode tip, and a proximal end of the conductive member is electrically connected to the control switch or the external power source.

8. The control handle of claim 7, wherein the proximal end of the conductive member is electrically connected to the first connector or the second connector; or the proximal end of the conductive member is electrically connected to the first cable or the second cable; or the proximal end of the conductive member is electrically connected to the external power source.

9. The control handle of claim 7, wherein
the conductive member is configured as a conductive wire, wherein a distal end of the conductive wire is fixedly connected to the electrode tip; or
the conductive member is configured as a conductive tube, wherein the conductive tube is integrally formed with the electrode tip, or the conductive tube is sleeved on the electrode tip, or the electrode tip is fixedly connected to a distal surface of the conductive tube.

10. The control handle of claim 4, wherein the operation member is movable relative to the housing in a radial direction of the housing.

11. The control handle of claim 10, wherein
the first connector comprises a connection piece and a first pin spaced apart from the connection piece;
the second connector comprises a base and a second pin, wherein the base is disposed corresponding to the first connector and located at one side of the connection piece away from the operation member, the first pin and the second pin are arranged at intervals on the base, the first pin is electrically connected to the distal end of the first cable, and the second pin is electrically connected to the distal end of the second cable; and two contact points are disposed on the base at intervals, wherein one of the two contact points is electrically connected to the first pin and the other of the two contact points is electrically connected to the second pin, and the triggering portion comprises the connection piece; and
the operation member is configured to drive, under the action of an external force, the connection piece to be in contact with the two contact points, to enable the control switch to be in the on-state.

12. The control handle of claim 11, wherein the control mechanism further comprises a fixing member, wherein the fixing member is insulated from the first connector and the second connector, and the base is disposed in the accommodation space via the fixing member; the fixing member comprises a fixing seat and a plurality of supporting legs supporting the fixing seat, wherein the plurality of supporting legs are fixed in the housing at intervals, and the fixing seat defines a groove for receiving the base and through-holes for allowing the first pin and the second pin to extend through, and wherein the through-holes are spaced apart from the plurality of supporting legs, and both the first pin and the second pin extend out of the fixing seat.

13. The control handle of claim 10, wherein the triggering portion comprises a free portion that is resilient, wherein the free portion is disposed on the first connector, and a free end of the free portion is movable within the accommodation space;
the second connector is located at one side of the first connector away from the operation member; and
the free portion is capable of driving, under an action of an external force, the free end of the free portion to move towards and be in contact with the second connector, to enable the control switch to be in the on-state.

14. The control handle of claim 13, wherein the first connector defines a through-hole at a middle portion of the first connector to form the free portion, wherein the free portion extends in an axial direction of the housing.

15. The control handle of claim 13, wherein the control mechanism further comprises a fixing member received in the accommodation space, wherein the fixing member is insulated from the first connector and the second connector;
an axis of the fixing member is coaxial with an axis of the housing, the fixing member defines a through-hole and an accommodation groove, wherein the through-hole extends through both ends of the fixing member that are arranged along the axis of the fixing member, and the accommodation groove has an opening facing a proximal end, the first connector extends through the through-hole, and the second connector is disposed in the accommodation groove; and
a distal end of the accommodation groove extends to a position corresponding to the free portion and is in communication with the through-hole, so that the free portion is operable to be in contact with the second connector.

16. The control handle of claim 10, wherein the first connector comprises a resilient piece and a conductive piece electrically connected to the resilient piece, wherein two ends of the conductive piece are both fixed to the resilient piece, the resilient piece is electrically connected to the first cable, the resilient piece defines a through-hole, the conductive piece is disposed corresponding to the through-hole of the resilient piece and between the resilient piece and the operation member, and the triggering portion comprises the conductive piece;
the second connector is disposed corresponding to the through-hole of the resilient piece and is electrically connected to the second cable, and the second connector is located at one side of the resilient piece away from the operation member; and
the operation member is configured to drive, under the action of an external force, a middle portion of the conductive piece to deform to be in contact with the second connector, to enable the control switch to be in the on-state.

17. The control handle of claim 16, wherein the conductive piece is arc-shaped, protrudes outwards towards the operation member, and extends in an axial direction of the housing; and
the second connector has a protruding portion configured to be electrically connected to the conductive piece, wherein the protruding portion extends in a circumferential direction of the housing.

18. The control handle of claim 17, wherein the control mechanism further comprises a fixing member received in the accommodation space, wherein the fixing member is insulated from the first connector and the second connector; and
the fixing member extends in the axial direction, wherein the fixing member is hollow to define an accommodation space for receiving the first connector and the second connector, the fixing member defines an opening, on a top of the fixing member, for allowing the pressing portion of the operation member to abut against and be in contact with the conductive piece, to enable the conductive piece to be in contact with the second connector.

19. The control handle of claim 18, wherein the fixing member defines a through-hole on a proximal end of the fixing member, a distal end of the resilient piece is connected to the fixing member via a fixing shaft, and a proximal end of the resilient piece extends through the through-hole of the fixing member; and
a proximal end of the second connector extends through the through-hole of the fixing member.

20. The control handle of claim 4, wherein the operation member is movable relative to the housing in an axial direction of the housing.

21. The control handle of claim 20, wherein the first connector has a first protrusion protruding towards the operation member, a proximal end of the first connector is electrically connected to the first cable, and a distal end of the first connector is electrically connected to the conductive member;
the second connector has a second protrusion corresponding to the first protrusion, wherein a proximal end of the second connector is electrically connected to the second cable, and the triggering portion comprises the first protrusion and the second protrusion; and
the first protrusion is disposed on a path along which the operation member is movable, wherein when the operation member moves to a position where the first protrusion is located, the pressing portion of the operation member abuts against the first protrusion, so that the first protrusion is deformed to be in contact with the second protrusion to enable the control switch to be in the on-state.

22. The control handle of claim 21, wherein the second connector is in a tubular shape, and defines an opening for allowing the operation member to move;
fitting members are respectively sleeved on two ends of the first connector, and the second connector is sleeved on an outer periphery of the fitting member, so that the first connector is received in an inner cavity of the second connector, and the first connector is insulated from the second connector via the fitting members.

23. The control handle of claim 2, comprising a fluid supply mechanism, wherein the fluid supply mechanism comprises a fluid supply tubular member disposed at the middle of the housing, the fluid supply tubular member extends into the accommodation space in a direction towards a distal end of the housing, an outer periphery of the fluid supply tubular member and an inner wall of the housing cooperatively define a space for allowing both the first cable and the second cable to extend through; and
both the first connector and the second connector are located at a distal end of the fluid supply tubular member and are spaced apart from the fluid supply tubular member, and the distal end of the fluid supply tubular member is in communication with a proximal end of a tube set extending into the accommodation space.

24. The control handle of claim 2, wherein the control handle comprises a fluid supply mechanism, wherein the fluid supply mechanism comprises a fluid supply tubular member disposed at the middle of the housing, wherein the fluid supply tubular member extends into the accommodation space and extends to a proximal end of the housing, and defines at a position corresponding to the operation member an opening for allowing the operation member to extend through, and the operation member is in interference fit with the opening of the fluid supply tubular member; and
the first connector and the second connector are received in the fluid supply tubular member, and part of the operation member is received in the fluid supply tubular member.

25. The control handle of claim 24, wherein the second connector is in snap-fit with the fluid supply tubular member; or a fixing member for fixing the first connector and the second connector is in snap-fit with the fluid supply tubular member.

26. The control handle of claim 24, wherein the control mechanism further comprises a protective film that is insulating and waterproof, wherein the protective film covers an outer periphery of the first connector and an outer periphery of the second connector and the operation member.

27. A puncture system, comprising an electrode tip, a tube set sleeved on the electrode tip, and the control handle of any one of claims 1 to 26, wherein a distal end of the control handle is fixedly connected to a proximal end of the tube set.
